# EUROPÄISCHE PATENTSCHRIFT

(11) **EP 1 137 794 B1**
(45) Veröffentlichungstag und Bekanntmachung des Hinweises auf die Patenterteilung: **05.03.2003**
(21) Anmeldenummer: 99957331.4
(22) Anmeldetag: 01.12.1999
(51) Int. Cl.: C12P 7/62, A61K 7/00

(54) **ENZYMATISCHE VERESTERUNG**
ENZYMATIC ESTERIFICATION
ESTERIFICATION ENZYMATIQUE

(30) Priorität: 10.12.1998 DE 19856948
(43) Veröffentlichungstag der Anmeldung: 04.10.2001
(73) Patentinhaber: Cognis Deutschland GmbH & Co. KG, 40589 Düsseldorf (DE)
(72) Erfinder: VONDERHAGEN, Anja, D-40223 Düsseldorf (DE)
(86) Internationale Anmeldenummer: EP9909378
(87) Internationale Veröffentlichungsnummer: WO00034501

(56) Entgegenhaltungen:
- EP-A- 0 229 400
- EP-A- 0 383 405
- EP-A- 0 519 727
- EP-A- 0 740 933
- WO-A-94/12652
- WO-A-98/55642
- WO-A-99/46397
- DE-A- 19 753 789
- GB-A- 2 272 904
- US-A- 5 147 791
- US-A- 5 478 910
- H. UYAMA ET AL.: "Enzymatic polymerization of dicarboxylic acid and glycol to polyester in solvent-free system" CHEMISTRY LETTERS, Bd. 12, Dezember 1998 (1998-12), Seiten 1285-1286, XP002134708 THE CHEMICAL SOCIETY OF JAPAN, JP
- KLINE ET AL: "Synthesis and characterization of aliphatic polyesters with hydrophilic pendant groups using enzymes" CHEMICAL ABSTRACTS + INDEXES,US,AMERICAN CHEMICAL SOCIETY. COLUMBUS, Bd. 20, Nr. 129, 16. November 1998 (1998-11-16), XP002106733 ISSN: 0009-2258
- S. KOBAYASHI ET AL.: "Dehydration polymerization in aqueous medium catalyzed by lipase" CHEMISTRY LETTERS, Bd. 11, November 1997 (1997-11), Seite 105 XP002134709 THE CHEMICAL SOCIETY OF JAPAN, JP
- BERKANE C ET AL: "LIPASE-CATALYZED POLYESTER SYNTHESIS ON ORGANIC MEDIUM. STUDY OF RING-CHAIN EQUILIBRIUM" MACROMOLECULES,US,AMERICAN CHEMICAL SOCIETY. EASTON, Bd. 30, Nr. 25, 15. Dezember 1997 (1997-12-15), Seiten 7729-7734, XP000727536 ISSN: 0024-9297

## Beschreibung

Die Erfindung betrifft ein Verfahren zur Herstellung von linearen Polyestem, aus aliphatische, substituierte aliphatische, aromatische und/oder substituierte aromatische Dicarbonsäuren und Polyolen in Gegenwart geringer Mengen eines niedrigsiedenden primären Alkohols, ohne Zusatz von Lösungsmittel und Lipase als Katalysator. Die linearen Polyester werden als Verdicker und Weichmacher in kosmetischen Zubereitungen vorgeschlagen.

### Stand der Technik

Die Veresterung von Dicarbonsäuren mit polyfunktionellen Alkoholen ist allgemein bekannt. Zum Beispiel wird in der GB-A-2 272 904 ein Verfahren zu Herstellung von linearen Polyestern aus aliphatische Dicarbonsäure und aliphatische Polyolen in Gegenwart einer Lipase und eines organische Lösungsmittel offenbart.

Die Veresterung von Dicarbonsäuren mit polyfunktionellen Alkoholen läßt sich chemisch nur sehr schwer steuern und liefert unter sehr hohen Temperaturen, d.h. über den Schmelzpunkten der Dicarbonsäuren, hochvemetzte Polyester. Die Lipase-katalysierte Umesterung von Dicarbonsäureestern führt hingegen unter milden Reaktionsbedingungen zur Bildung linearer Polyester. Im Gegensatz dazu ist man bei der Veresterung der freien Dicarbonsäuren auf die Verwendung eines Lösungsmittels angewiesen, da die optimalen Reaktionstemperaturen der Lipase unterhalb der Schmelztemperaturen der Dicarbonsäuren liegen.
Da die freien Dicarbonsäuren technisch leichter zugänglich sind als deren Ester, bestand die Aufgabe der vorliegenden Erfindung darin ein neues Verfahren zur Verfügung zu stellen, welches lineare Polyester aus Dicarbonsäuren und Polyolen unter milden Bedingungen ohne Verwendung von Lösungsmittel in quantitativen Ausbeuten und günstigen Reaktionszeiten zugänglich macht.

### Beschreibung der Erfindung

Ein Gegenstand der vorliegenden Erfindung ist ein Verfahren zur Herstellung von linearen Polyestern, bei dem man aliphatische, substituierte aliphatische, aromatische und/oder substituierte aromatische Dicarbonsäuren mit 2 bis 10 Kohlenstoffatomen und Polyolen mit 2 bis 15 Kohlenstoffatomen und mindestens zwei primären Hydroxylgruppen in Gegenwart geringer Mengen eines niedrigsiedenden primären Alkohols, ohne Zusatz von Lösungsmittel und Lipase als Katalysator umsetzt.

Ein weiterer Gegenstand der Erfindung betrifft die Verwendung linearer Polyester, die man durch Umsetzung von aliphatische, substituierte aliphatische, aromatische und/oder substituierte aromatische Dicarbonsäuren mit 2 bis 10 Kohlenstoffatomen und Polyolen mit 2 bis 15 Kohlenstoffatomen und mindestens zwei primären Hydroxylgruppen in Gegenwart geringer Mengen eines niedrigsiedenden primären Alkohols, ohne Zusatz von Lösungsmittel und Lipase als Katalysator erhält, als Verdicker und Weichmacher in kosmetischen Zubereitungen.

Überraschenderweise wurde gefunden, daß sich lineare Polyester aus Dicarbonsäuren und Polyolen in Gegenwart geringer Mengen eines niedrigsiedenden primären Alkohols und Lipase als Katalysator erstmalig ohne Zusatz von Lösungsmittel herstellen lassen. Hierbei zeichnet sich die Veresterung durch milde Reaktionsbedingungen, günstige Reaktionszeiten und quantitative Ausbeuten aus. Die Erfindung schließt die Erkenntnis mit ein, daß bei Einsatz von Dicarbonsäuremischungen enthaltend Hydroxydicarbonsäuren auch lineare Polyester erhalten werden, die vorteilhafterweise wasserlöslich sind.

### Dicarbonsäuren

Zur Herstellung von Polyestern werden aliphatische, substituierte aliphatische, aromatische und/oder substituierte aromatische Dicarbonsäuren mit 2 bis 10, vorzugsweise 3 bis 9 und insbesondere 4 bis 6 Kohlenstoffatomen eingesetzt. Als aliphatische Dicarbonsäuren werden beispielsweise Oxalsäure, Malonsäure, Bernsteinsäure und Adipinsäure, Azelainsäure, Dodecandisäure und Brassylsäure und als aromatische Dicarbonsäuren Phthalsäure, Isophthalsäure und Terephthalsäure verwendet. Als substituierte Dicarbonsäuren können die Aminodisäuren, vorzugsweise Glutaminsäure und Hydroxydicarbonsäuren mit mindesten zwei primären Hydroxylgruppen, vorzugsweise Apfelsäure, Citronensäure, Glutaconsäure und Glucarsäure und insbesondere Weinsäure, eingesetzt werden. Bevorzugt werden aliphatische unverzweigte Dicarbonsäuren und Hydroxydicarbonsäuren verwendet.
Außerdem können Mischungen aus Dicarbonsäuren eingesetzt werden, wobei mindestens eine Dicarbonsäure eine Hydroxydicarbonsäure mit mindestens zwei primären Hydroxylgruppen, vorzugsweise Apfelsäure, Citronensäure, Glutaconsäure und Glucarsäure und insbesondere Weinsäure, darstellt. Man wählt ein molares Verhältnis Dicarbonsäure : Hydroxydicarbonsäure von 1:10 bis 10:1, vorzugsweise 1:5 bis 5:1, insbesondere 1:2 bis 1:1.

### Polyole

Polyole, die im Sinne der Erfindung in Betracht kommen, besitzen vorzugsweise 2 bis 15 Kohlenstoffatome, vorzugsweise 3 bis 7 und insbesondere 4 bis 6 und mindestens 2, vorzugsweise höchstens 6 und insbesondere 3 Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Alkylenglykole werden vorzugsweise Propylenglycol, Butylenglycol, Hexylenglycol sowie Polyethylenglycole mit einem durchschnittlichen Molekulargewicht im Bereich von 100 bis 1.000 Dalton eingesetzt. Insbesondere geeignete Polyole sind Glycerin, Sorbitol, Sorbitan, Trimethylolpropan, Pentaerythritol und 2-Amino-1,3-propandiol. Deren Oligomere, wie beispielsweise Dimere, Trimere Tetramere, Pentamere und/oder Hexamere, können ebenfalls verwendet werden.

### Niedrigsiedende primäre Alkohole

Niedrigsiedende primäre Alkohole, die im Sinne dieser Erfindung in Betracht kommen, sind Alkohole die einen Siedepunkt unterhalb von 100 °C besitzen. Vorzugsweise werden Methanol, Isopropanol und Propanol und insbesondere Ethanol eingesetzt.

### Lipasen

Zur Herstellung der erfindungsgemäßen linearen Polyole werden als Katalysator Lipasen verwendet, die sich von Candida cylindracea, Candida lipolytica, Candida rugosa, Candida antarctica B, Candida utilis, Chromobacterium viscosum, Geotrichum viscosum, Geotrichum candidum, Mucor javanicus, Mucor miehei, Porcine pancreas, Pseudomonas species, Pseudomodas fluorescens, Pseudomodas cepacia, Rhizomucor miehei, Rhizopus arrhizus, Rhizopus delemar, Rhizopus niveus, Rhizopus oryzae, Aspergillus niger, Penicillium roquefortii, Penicillum cambertii, Pseudodomas fluorescens oder einer Esterase aus Bacillus sp., Bacillus thermoglucosidasius, Mucor miehei, Horse liver, Saccharomyces cerevisiae, Pig liver ableiten. Hierbei können sowohl die Lipasen alleine als auch eine Kombination verwendet werden. Die Lipasen werden in Mengen eingesetzt, die eine ausreichende Katalyse der erfindungsgemäßen Poly-Veresterung bewirken und zu einem gewünschten Molekulargewicht der lineraren Polyester führen. Vorzugsweise werden Lipasen verwendet, die sich aus Mucor miehei und Aspergillus niger ableiten. Insbesondere setzt man Novozym® 388 L (Rhizomucor miehei Lipase, frei), Lipozym® IM (Rhizomucor miehei Lipase, immobilisiert), Novozym® 735 L (Candida antarctica B Lipase, frei), Novozym® 525 L (Candida antarctica B Lipase, frei) und/oder Novozym® 435 (Candida antarctica B Lipase, immobilisiert) ein, welche Produkte der Firma Novo Nordisk (Dänemark) darstellen. Bevorzugt werden die Lipasen in Mengen von 0,01 bis 15 und insbesondere von 1 bis 10 Gew.-% ― bezogen auf die Dicarbonsäure ― eingesetzt.

### Herstellungsverfahren

Die linearen Polyole werden erfindungsgemäß durch Umsetzung von Dicarbonsäuren bzw. Mischungen von Dicarbonsäuren mit einer Hydroxydicarbonsäure mit Polyolen in Gegenwart geringer Mengen eines niedrigsiedenden primären Alkohols ohne Zusatz von Lösungsmittel und Lipase als Katalysator erhalten. Der freiwerdende niedrigsiedende Alkohol wird vorzugsweise aus dem Reaktionsgemisch entfernt werden. Die Reaktion erfolgt vorzugsweise bei Temperaturen im Bereich von 50 bis 90, insbesondere 70 °C und in der Regel bei Drücken von 0 bis 1013, vorzugsweise 0,01 bis 500 und insbesondere 10 bis 250 mbar. Die Reaktionszeit beträgt 8 Stunden bis 4 Tage, vorzugsweise 24 bis 48 Stunden. Zur Herstellung der erfindungsgemäßen Polyester wählt man ein molares Verhältnis Dicarbonsäuren : Polyole von 2:1 bis 0,5:1, vorzugsweise 1,2:1 bis 0,8:1. Die niedrigsiedenden primären Alkohole werden in einem 0,1 bis 5, vorzugsweise 0,2 bis 2 und insbesondere 0,3 bis 1 molaren Überschuß ― bezogen auf die Dicarbonsäure ― eingesetzt. Durch dieses Verfahren können Polyester mit einem Molekulargewicht von 483 bis 10.000 (bestimmt aus GPC-Daten und Eichung mit Polyethylenglycol) bzw. von 22.000 (bestimmt aus GPC-Daten und Eichung mit Polystyrol) hergestellt werden.

### Gewerbliche Anwendbarkeit

Die erfindungsgemäßen linearen Polyester können zur Herstellung von kosmetischen Zubereitungen, wie beispielsweise Haarshampoos, Haarlotionen, Schaumbäder, Cremes, Gele, Lotionen, alkoholische und wäßrig/alkoholische Lösungen, Emulsionen, Öle, Wachs/Fett-Massen, Stiftpräparate, Puder oder Salben dienen. Diese Mittel können ferner als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quellmittel, UV-Lichtschutzfaktoren, Antioxidantien, Hydrotrope, Konservierungsmittel, Insektenrepellentien, Selbstbräuner, Solubilisatoren, Parfümöle, Farbstoffe, keimhemmende Mittel und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkohoten, Ester von verzweigten C₆-C₁₃-Carbonsäuren mit linearen C₆-C₂₂ Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daβ sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohtenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethytenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte VinylpyrrolidonNinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/ Isobomylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/Acrylat-Copolymere,Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Eine detaillierte Übersicht über geeignete flüchtige Silicone findet sich zudem von Todd et al. in **Cosm.Toil. 91, 27 (1976)**.

Typische Beispiele für **Fette** sind Glyceride, als **Wachse** kommen u.a. Bienenwachs, Carnaubawachs, Candelillawachs, Montanwachs, Paraffinwachs, hydriertes Ricinusöle, bei Raumtemperatur feste Fettsäureester oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden.

Unter **biogenen Wirkstoffen** sind beispielsweise Tocopherol, Tocopherolacetat, Tocopherolpatmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.
Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc. Cosm.Chem. 24, 281 (1973)**]. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)**]. Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw, deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B.

Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester,
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben;
- Propan-1,3-dione, wie z.B. 1-(4-tert.Butylphenyl)-3-(4'-methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. In Sonnenschutzmitteln werden bevorzugt sogenannte Mikro- oder Nanopigmente eingesetzt. Vorzugsweise wird mikronisiertes Zinkoxid verwendet.

Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, η-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, αGlycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insekten-Repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4-chlorphenyl-biguanido)-hexan) oder TCC (3,4,4'-Trichlorcarbanilid). Auch zahlreiche Riechstoffe und etherische Öle weisen antimikrobielle Eigenschaften auf. Typische Beispiele sind die Wirkstoffe Eugenol, Menthol und Thymol in Nelken-, Minz- und Thymianöl. Ein interessantes natürliches Deomittel ist der Terpenalkohol Farnesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei etwa 0,1 bis 2 Gew.-% - bezogen auf den auf den Feststoffanteil der Zubereitungen.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Beispiele

### Beispiel 1:

18,2 g (0,1 mol) Azelainsäure wurden mit 4,6 g Ethanol (0,1 mol) und 9,2 g (0,1 mol) Glycerin versetzt und die Reaktion mit 1,4 g (5 Gew.-%) Novozym® 435 (immobilisierte Lipase aus Candida antarctica B, Firma Novo Nordisk) gestartet. Nachdem die Reaktionsmischung 4 Stunden bei 75 °C und 1013 mbar gerührt wurde, reduzierte man zum kontinuierlichen Abdestillieren des Ethanols den Druck auf 500 mbar und später auf 50 mbar. Nach 24 Stunden wurde das immobilisierte Enzym durch Filtration entfernt. Man erhielt ca. 25 g einer gelben, viskosen Flüssigkeit mit einem Molekulargewicht (bestimmt aus GPC-Daten und Eichung mit Polysterol) von ca. 1200, die Säurezahl liegt bei ca. 50.

### Beispiele 2 und 3:

Die gleiche Umsetzung wie in Beispiel 1 wurde mit 6,0 g (0,1 mol) Isopropanol oder Propanol anstelle von Ethanol durchgeführt, wobei man vergleichbare Ergebnisse erhielt.

### Beispiel 4:

150,1 g (1 mol) L-(+)-Weinsäure und 188,2 g (1 mol) Azelainsäure wurden mit 46,1 (1 mol) Ethanol und 184,2 g (1 mol) Glycerin versetzt und die Reaktion mit 26 g (5 Gew.-%) Novozym® 435 (immobilisierte Lipase aus Candida antarctica B, Firma Novo Nordisk) gestartet Nachdem die Reaktionsmischung 6 Stunden bei 80 °C und 1013 mbar gerührt wurde, reduzierte man langsam zum kontinuierlichen Abdestillieren des Ethanols den Druck auf 200 mbar. Die Reaktionszeit betrug 3 Tage. Anschließend wurde das immobilisierte Enzym durch Filtration entfernt. Man erhielt ca. 400 g einer klaren viskosen Flüssigkeit mit einem Molekulargewicht (bestimmt aus GPC-Daten und Eichung mit Polysterol) von ca. 1500, die Säurezahl liegt bei ca. 93. Das Produkt ist wasserlöslich.

### Beispiel 5:

150,1 g (1 mol) L-(+)-Weinsäure wurden mit 46,1 (1 mol) Ethanol und 92,1 g (1 mol) Glycerin versetzt und die Reaktion mit 12,1 g (5 Gew.-%) Novozym® 435 (immobilisierte Lipase aus Candida antarctica B, Firma Novo Nordisk) gestartet. Nachdem die Reaktionsmischung 5 Stunden bei 80 °C und 1013 mbar gerührt wurde, reduzierte man innerhalb von 24 Stunden zum kontinuierlichen Abdestillieren des Ethanols den Druck auf 230 mbar und nach weiteren 24 Stunden auf 50 mbar. Anschließend wurde das immobilisierte Enzym durch Filtration entfernt. Man erhielt ca. 200 g einer viskosen Flüssigkeit mit einem Molekulargewicht (bestimmt aus GPC-Daten und Eichung mit Polysterol) von ca. 1100, die Säurezahl liegt bei ca. 122.

## Patentansprüche

1. Verfahren zur Herstellung von linearen Polyestern, bei dem man aliphatische, substituierte aliphatische, aromatische und/oder substituierte aromatische Dicarbonsäuren mit 2 bis 10 Kohlenstoffatomen und Polyolen mit 2 bis 15 Kohlenstoffatomen und mindestens zwei primären Hydroxylgruppen in Gegenwart geringer Mengen eines niedrigsiedenden primären Alkohols, ohne Zusatz von Lösungsmittel, und Lipase als Katalysator umsetzt.

2. Verfahren nach dem Anspruch 1, **dadurch gekennzeichnet, daß** man Polyole ausgewählt aus der Gruppe, die gebildet wird von Glycerin, Alkylenglycolen, technischen Oligoglyceringemischen, Methylolverbindungen, Niedrigalkylglucosiden, Zuckeralkoholen, Zuckern und Aminozuckern einsetzt.

3. Verfahren nach den Ansprüchen 1 und/oder 2, **dadurch gekennzeichnet, daß** man als Lipase Candida antarctica B oder Rhizomucor miehei verwendet.

4. Verfahren nach mindestens einem der Ansprüche 1 bis 3, **dadurch gekennzeichnet, daß** man Mischungen von Dicarbonsäuren einsetzt, in dem mindestens eine Dicarbonsäure eine Hydroxydicarbonsäure mit mindestens zwei primären Hydroxylgruppen ist.

5. Verfahren nach mindestens einem der Ansprüche 1 bis 4, **dadurch gekennzeichnet, daß** man Mischungen von Dicarbonsäuren einsetzt, in dem mindestens eine Dicarbonsäure Weinsäure ist.

6. Verfahren nach mindestens einem der Ansprüche 1 bis 5, **dadurch gekennzeichnet, daß** man Mischungen von Dicarbonsäuren im molaren Verhältnis von 1:10 bis 10 :1 einsetzt.

7. Verfahren nach mindestens einem der Ansprüche 1 bis 6, **dadurch gekennzeichnet, daß** man die Dicarbonsäuren mit Polyolen im molaren Verhältnis von 2:1 bis 0,5:1 einsetzt.

8. Verfahren nach mindestens einem der Ansprüche 1 bis 7, **dadurch gekennzeichnet, daß** man die Dicarbonsäuren mit Polyolen im molaren Verhältnis von 1,2:1 bis 0,8:1 einsetzt.

9. Verfahren nach mindestens einem der Ansprüche 1 bis 8, **dadurch gekennzeichnet, daß** man den niedrigsiedenden primären Alkohol in einem 0,1 bis 5 molaren Überschuß - bezogen auf die Dicarbonsäure - einsetzt.

10. Verfahren nach mindestens einem der Ansprüche 1 bis 9, **dadurch gekennzeichnet, daß** man die Umsetzung bei Drücken von 0,01 bis 500 mbar durchführt.

11. Verfahren nach mindestens einem der Ansprüche 1 bis 10, **dadurch gekennzeichnet, daß** man die Umsetzung bei Drücken von 10 bis 250 mbar durchführt.

12. Verwendung von linearen Polyestern, erhältlich durch Umsetzung von aliphatische, substituierte aliphatische, aromatische und/oder substituierte aromatische Dicarbonsäuren mit 2 bis 10 Kohlenstoffatomen und Polyolen mit 2 bis 15 Kohlenstoffatomen und mindestens zwei primären Hydroxylgruppen in Gegenwart geringer Mengen eines niedrigsiedenden primären Alkohols, ohne Zusatz von Lösungsmittel, und Lipase als Katalysator, als Verdicker und Weichmacher in kosmetischen Zubereitungen.

## Claims

1. A process for the production of linear polyesters in which aliphatic, substituted aliphatic, aromatic and/or substituted aromatic dicarboxylic acids containing 2 to 10 carbon atoms and polyols containing 2 to 15 carbon atoms and at least two primary hydroxyl groups are reacted in the presence of small quantities of a low-boiling primary alcohol and lipase as catalyst without the addition of solvents.

2. A process as claimed in claim 1, **characterized in that** polyols selected from the group consisting of glycerol, alkylene glycols, technical oligoglycerol mixtures, methylol compounds, lower alkyl glucosides, sugar alcohols, sugars and aminosugars are used.

3. A process as claimed in claims 1 and/or 2, **characterized in that** Candida antarctica B or Rhizomucor miehei is used as the lipase.

4. A process as claimed in at least one of claims 1 to 3, **characterized in that** mixtures of dicarboxylic acids in which at least one dicarboxylic acid is a hydroxydicarboxylic acid containing at least two primary hydroxyl groups are used.

5. A process as claimed in at least one of claims 1 to 4, **characterized in that** mixtures of dicarboxylic acids in which at least one dicarboxylic acid is tartaric acid are used.

6. A process as claimed in at least one of claims 1 to 5, **characterized in that** mixtures of dicarboxylic acids in a molar ratio of 1:10 to 10:1 are used.

7. A process as claimed in at least one of claims 1 to 6, **characterized in that** the dicarboxylic acids and polyols are used in a molar ratio of 2:1 to 0.5:1.

8. A process as claimed in at least one of claims 1 to 7, **characterized in that** the dicarboxylic acids and polyols are used in a molar ratio of 1.2:1 to 0.8:1.

9. A process as claimed in at least one of claims 1 to 8, **characterized in that** the low-boiling primary alcohol is used in a 0.1 to 5 molar excess, based on the dicarboxylic acid.

10. A process as claimed in at least one of claims 1 to 9, **characterized in that** the reaction is carried out under pressures of 0.01 to 500 mbar.

11. A process as claimed in at least one of claims 1 to 10, **characterized in that** the reaction is carried out under pressures of 10 to 250 mbar.

12. The use of linear polyesters obtainable by reaction of aliphatic, substituted aliphatic, aromatic and/or substituted aromatic dicarboxylic acids containing 2 to 10 carbon atoms and polyols containing 2 to 15 carbon atoms and at least two primary hydroxyl groups in the presence of small quantities of a low-boiling primary alcohol and lipase as catalyst without the addition of solvents as thickeners and softeners in cosmetic preparations.

## Revendications

1. Procédé de préparation de polyesters linéaires, dans lequel on fait réagir des acides dicarboxyliques aliphatiques, aliphatiques substitués, aromatiques et/ou aromatiques substitués, ayant de 2 à 10 atomes de carbone, et des polyols ayant de 2 à 15 atomes de carbone et au moins deux groupes hydroxyle primaires, en présence d'un alcool primaire à bas point d'ébullition sans ajout de solvant, et en présence de lipase comme catalyseur.

2. Procédé selon la revendication 1,
**caractérisé en ce qu'**
on met en oeuvre des polyols choisis dans le groupe formé du glycérol, des alkylèneglycols, des mélanges industriels d'oligoglycérols, des composés méthyloliques, des (alkyl inférieur) glucosides, des alcools de sucre, des sucres et des aminosucres.

3. Procédé selon les revendications 1 et/ou 2,
**caractérisé en ce qu'**
on utilise comme lipase Candida antarctica B ou Rhizomucor miehei.

4. Procédé selon au moins une des revendications 1 à 3,
**caractérisé en ce qu'**
on met en oeuvre des mélanges d'acides dicarboxyliques dans lesquels au moins un acide dicarboxylique est un acide hydroxydicarboxylique ayant au moins deux groupes hydroxyle primaires.

5. Procédé selon au moins l'une des revendications 1 à 4,
**caractérisé en ce qu'**
on met en oeuvre des mélanges d'acides dicarboxyliques dans lesquels au moins un acide dicarboxylique est l'acide tartarique.

6. Procédé selon au moins l'une des revendications 1 à 5,
**caractérisé en ce qu'**
on met en oeuvre des mélanges d'acides dicarboxyliques avec des polyols dans un rapport molaire de 1 : à 10 :1.

7. Procédé selon au moins une des revendications 1 à 6,
**caractérisé en ce qu'**
on met en oeuvre des acides dicarboxyliques avec des polyols dans un rapport molaire de 2 :1 à 0,5 :1.

8. Procédé selon au moins une des revendications 1 à 7,
**caractérisé en ce qu'**
on met en oeuvre les acides carboxyliques avec des polyols dans un rapport molaire de 1,2 :1 à 0,8 :1.

9. Procédé selon au moins une des revendications 1 à 8,
**caractérisé en ce qu'**
on met en oeuvre l'alcool primaire à bas point d'ébullition en un excès 0,1 à 5 molaire - rapporté à l'acide dicarboxylique.

10. Procédé selon au moins une des revendications 1 à 9,
**caractérisé en ce qu'**
on effectue la réaction à des pressions allant de 0,01 à 500 mbars.

11. Procédé selon au moins une des revendications 1 à 10,
**caractérisé en ce qu'**
on effectue la réaction à des pressions allant de 10 à 250 mbars.

12. Utilisation de polyesters linéaires accessibles par réaction d'acides dicarboxyliques aliphatiques, aliphatiques substitués, aromatiques et/ou aromatiques substitués ayant de 2 à 10 atomes de carbone, et de polyols ayant de 2 à 15 atomes de carbone et au moins deux groupes hydroxyle primaires, en présence de faibles quantités d'un alcool primaire à bas point d'ébullition - sans ajout de solvant - et d'une lipase comme catalyseur, en tant qu'agent épaississant et d'agents assouplissants dans des préparations cosmétiques.
